# EUROPEAN PATENT APPLICATION

(11) **EP 4 726 693 A1**
(43) Date of publication of application: **15.04.2026**
(21) Application number: 24797460.3
(22) Date of filing: 26.04.2024
(51) Int. Cl.: G08B 17/117, G08B 5/36, G08B 3/10, G08B 25/00, G08B 25/14, G01N 33/00, G06Q 50/10, A62C 13/76, H02B 13/025, H04L 61/5007

(54) **DISTRIBUTION BOARD FIRE PREVENTION MANAGEMENT SYSTEM**

(30) Priority: 27.04.2023 KR 20230055227
(71) Applicant: Lee, Seoung Choul, Daejeon 34679 (KR)
(72) Inventor: Lee, Seoung Choul, Daejeon 34679 (KR)
(74) Representative: Spengler, Robert
(86) International application number: PCT/KR2024/005666
(87) International publication number: WO 2024/225806

(57) **Abstract**

A fire prevention management system for a distribution board according to the present invention comprises; a distribution board consisting of multiple metal enclosures; a target gas sensing member installed inside a metal enclosure; a monitoring member installed outside the metal enclosure that analyzes signals from the target gas sensing member; and a communication member that, based on the analyzed concentration and occurrence location of the target gas, issues step-by-step alarms, sends signals, induces shutdown of an installed equipment, and transmits abnormal status.

## Description

### Field of the Invention

The present invention relates to a fire prevention management system for a distribution board, and more particularly, to a system that detects target gases according to their concentration and sensing location, and issues stepwise alarms or performs control to prevent fire in the distribution board.

### Description of the Related Art

A distribution board is a facility that receives extra-high voltage or high voltage power from a power supplier and transforms it into a suitable voltage for a receiving facility. A 'board' is called 'panel' and is used interchangeably. Various types of power components such as switches, circuit breakers, transformers, current transformers, and protective relays are installed inside an enclosure, and are used as high-voltage distribution panels, low-voltage distribution panels, motor control panels, and distribution panels.

In facilities such as hospitals, power plants, substations, and factories where multiple distribution boards are installed, maintaining a stable power supply is critical. However, accidents such as leakage, discharge, fire, and condensation may occur due to deterioration of conductors or insulators, requiring strict monitoring.

Electrical fires account for more than 20% of all fires, and more than 80% of electrical fires are caused by deterioration of conductors or insulators, resulting in disconnection, short-circuiting, or poor contact.

Conventional protective relays detect overcurrent and cut off power exceeding a predetermined limit but cannot detect fire precursors that occur within the allowable current range.

Conventional techniques for preventing fires in distribution panels have involved monitoring by installing cameras, arc detectors, ultrasonic detectors, and electromagnetic wave detectors inside the panel. However, due to the structure of the distribution panel, numerous devices are installed in a narrow space, and they act as obstacles to or interfere with light or electromagnetic wave transmission, making it difficult to accurately detect warning signs of fire.

### DETAILED DESCRIPTION OF THE INVENTION

### Technical subjects

In order to solve the above problems, the present invention provides a fire prevention management system for a distribution board that detects target gas before ignition temperature and issues an alarm or controls it in multiple steps, regardless of problems occurring outside of the allowable capacity and interference environments of installed devices.

### Means of problem solving

A fire prevention management system for a distribution board according to the present invention comprises; a distribution board consisting of multiple metal enclosures; a target gas sensing member installed inside a metal enclosure; a monitoring member installed outside the metal enclosure that analyzes signals from the target gas sensing member; and a communication member that, based on the analyzed concentration and occurrence location of the target gas, issues step-by-step alarms, sends signals, induces shutdown of an installed equipment, and transmits abnormal status.

The target gas sensing member includes a first gas inlet for introducing target gas from a first occurrence location and a second gas inlet for introducing target gas from a second occurrence location.

The target gas sensing member includes an air injection member and an exhaust member that forcibly circulate air to discharge residual detected target gas and detect newly introduced gas in real-time.

The target gas sensing member is arranged in multiples within a single metal enclosure at an equal target gas diffusion distance.

The monitoring member determines a severity and controls it in the following steps: a first step is to issue a warning using a warning light or buzzer; a second step is to issue an emergency alarm by linking with the emergency bell of the indoor fire extinguisher; a third step is to send a text message to a manager and fire department; a fourth step is to forcibly cut off power to the equipment; and a fifth step is to operate a fire extinguisher. When multiple target gas sensing members are used, the occurrence location of the target gas is identified by the MAC address of the sensing member.

Alternatively, the occurrence location can be identified by the ID of the sensing member.

### The effect of the invention

Conventional fire prevention management systems using heat detection, are detection or electromagnetic wave detection have limitations in accurately detecting fires due to the numerous devices installed inside the distribution panel that act as obstacles. However, the fire prevention management system for the distribution panel according to the present invention detects fires by the diffusion of target gas generated before ignition, so it is less affected by interference from obstacles and can accurately and quickly manage fires before ignition in real time.

Furthermore, while conventional systems require one monitoring member per a sensor, the present invention provides an algorithm that monitors multiple distribution board sensors with a single monitoring member, reducing installation costs.

Even when multiple sensing members are used, the use of MAC addresses or IDs enables monitoring of numerous fire scenarios by combining target gas occurrence locations with gas concentrations.

### BRIEF DISCRIPTION OF THE DRAWINGS

FIG. 1 is a schematic diagram of a fire prevention management system for a distribution board according to the present invention.
FIG. 2 is a pyrolysis gas chromatography of an insulation used in electrical wires according to the present invention.
FIG. 3 is a pyrolysis gas chromatography of tubes used in terminal blocks according to the present invention.
FIG. 4 illustrates an embodiment of detecting according to target gas occurrence location using a gas flow switching member of the sensing member according to the present invention.
FIG. 5 is a detection circuit diagram of the sensor member according to the present invention.
FIG. 6 is a pictogram showing control in five steps according to the analysis results of the monitoring member.
FIG. 7 is a five-step control logic circuit diagram according to the present invention.

### DESCRIPTION OF THE PREFERRED EMBODIMENT

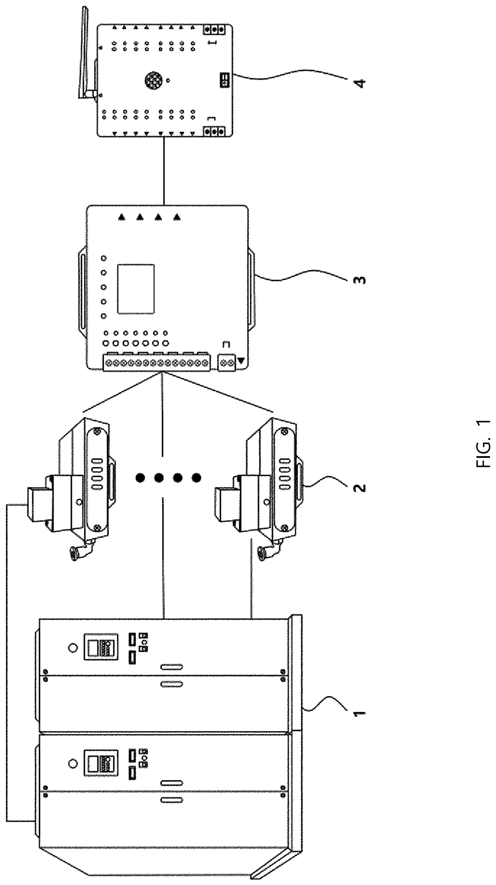

Fig. 1 shows a schematic diagram of a fire prevention management system for a distribution board according to the present invention, comprising; a distribution board (1) consisting of multiple metal enclosures; a target gas sensing member (2) installed inside a metal enclosure; a monitoring member (3) installed outside the metal enclosure for analyzing signals from the target gas sensing member; and a communication member (4) for sending step-by-step alarm or control signals based on the analyzed concentration and occurrence location of a target gas.

The distribution board is a facility that receives extra-high voltage or high voltage power from a power supplier and transforms it into suitable voltage for the consumer, and is equipped inside the enclosure with various power equipment such as switches, circuit breakers, transformers, current transformers, and protective relays, and is utilized as a high-voltage distribution board, low-voltage distribution board, motor control panel, and distribution board.

A deterioration and cause of fire in the distribution board occur when the temperature of conductor tubes or insulation coverings used in the distribution board rises, generating target gases before a ignition point and causing a reduction in weight.

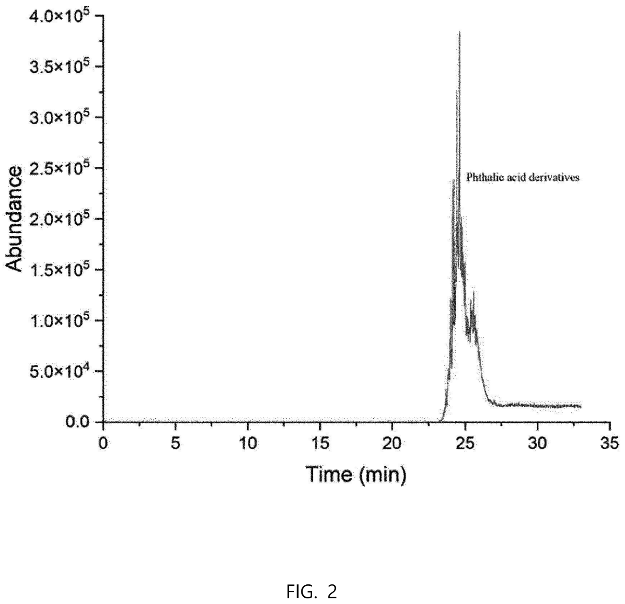

Fig. 2 shows the pyrolysis gas chromatography of the insulation used in electrical wires, in which phthalate-based target gas is generated.

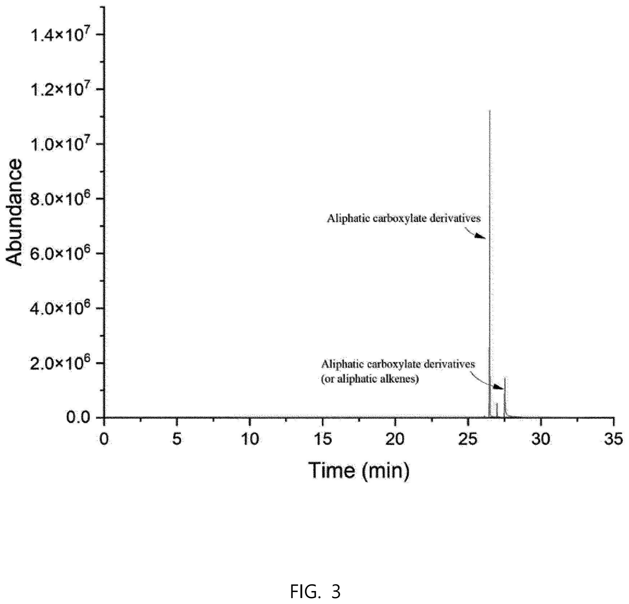

Fig. 3 shows the pyrolysis gas chromatography of the tube used in a terminal block, in which carboxylate-based target gas is generated. Phthalates and carboxylates are additives used as plasticizers or lubricants for the insulation or the tube. The present invention uses phthalates or carboxylates as target gases to provide fire prevention management, such as issuing warnings before an occurrence of ignition fires.

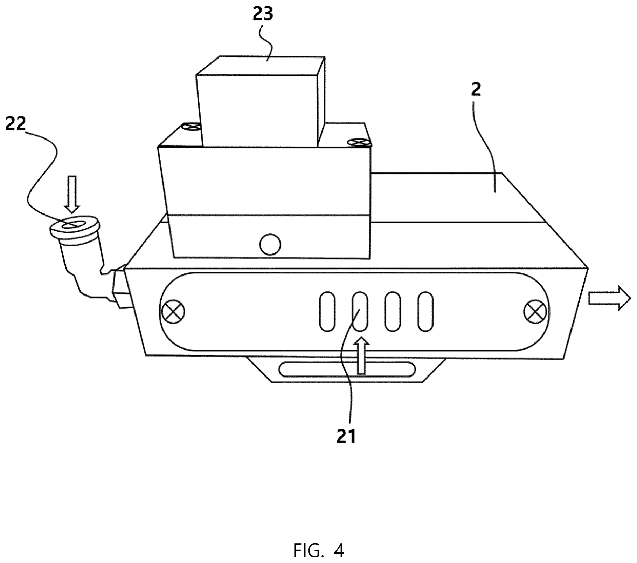

If the metal enclosure is small enough to be fully monitored by a single target gas sensing member, only one target gas sensing member is used. In such a case, a gas selection switching member (23) of the sensing member as shown Fig. 4 is used to periodically open and close a first gas inlet (21) and a second gas inlet (22) to monitor the target gas according to the occurrence location or distance. The gas selection switching member can operate automatically or manually.

Since the target gas is slightly heavier than air at room temperature and diffuses slowly, a fan is installed in the target gas sensing member to quickly draw the target gas into the sensor. A first occurrence location gas refers to the target gas generated in the nearby vicinity of the target gas sensing member, enabling monitoring of the internal environment of specific parts of relays and devices other than a main conductor and power supply equipment in the distribution board. A second occurrence location gas may be the target gas generated at a distant location from the target gas sensing member, such as from a submersible motor, and introduced via a tube or duct.

To reduce detection error of the target gas sensing member, captured target gas is forcibly discharged using an exhaust fan so that it does not remain inside the sensor, and fresh air or purification gas is introduced through a purification air injection member to refresh the sensor, thereby enabling continuous real-time monitoring.

In another embodiment of the present invention, when the metal enclosure is large and the monitoring area is wide, the target gas generated in corners or distant locations where detection by convectional diffusion of gas generated due to temperature rise is difficult may be delayed in being detected. To prevent this, multiple target gas sensing members may be arranged within a single metal enclosure at equal target gas diffusion distances.

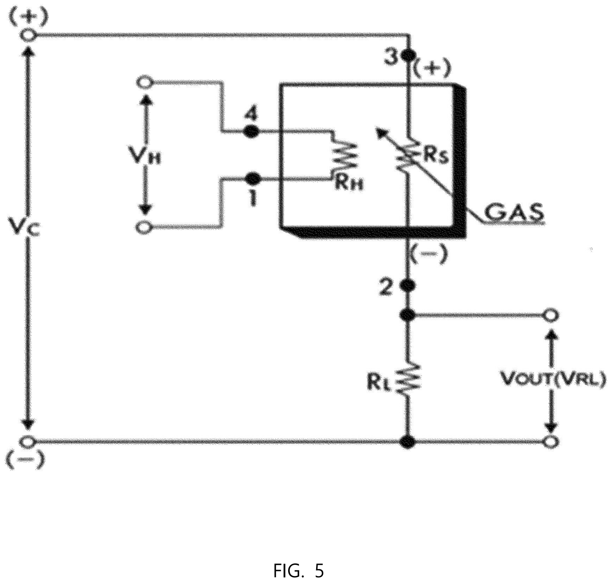

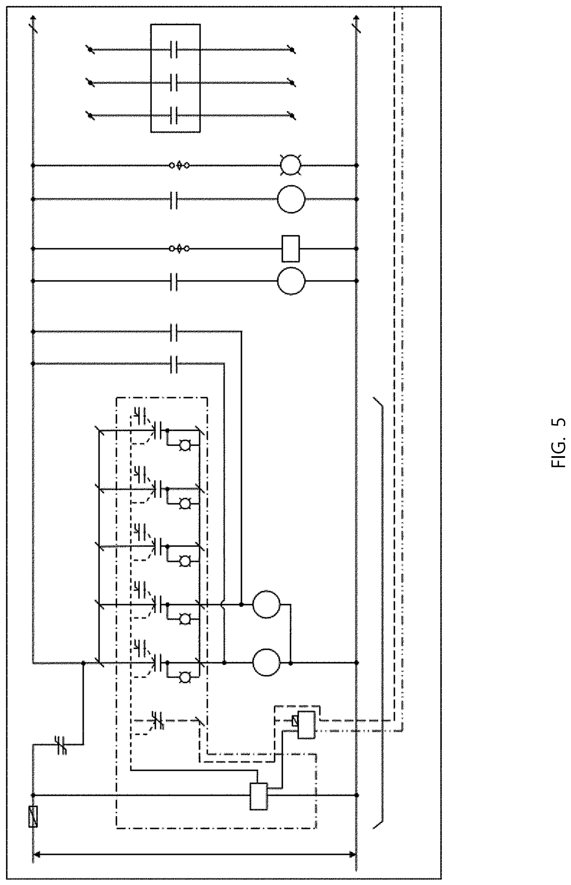

Fig. 5 shows a detection circuit of the target gas sensing member, in which a sensing resistor (R_{S}) varies according to a gas concentration and is output as a voltage (Vout) to a load (R_{L}). To reduce detection error, a resistive heater (R_{H}) is installed to volatilize the target gas attached to the sensing resistor (R_{S}).

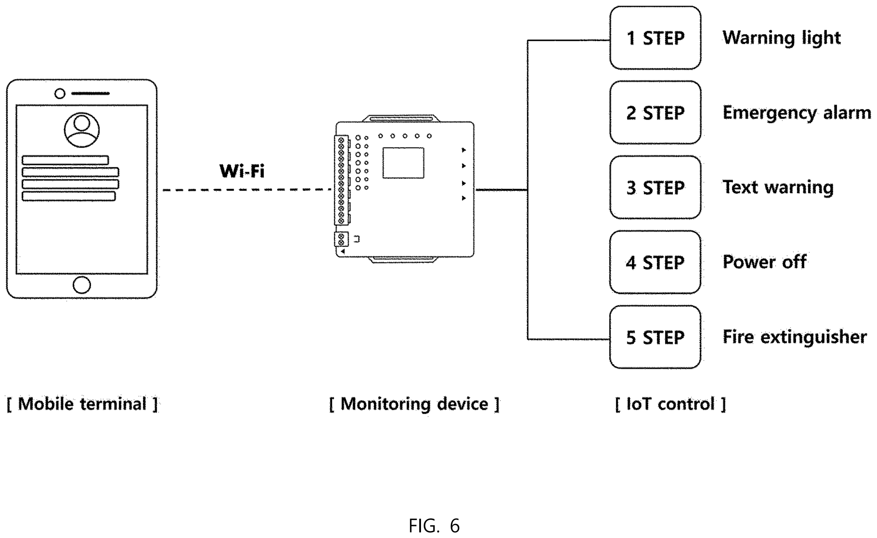

Fig. 6 is a pictogram of five-step control according to an analysis result of the monitoring member, and Fig. 7 is a circuit diagram of the five- step control logic. A resistance value detected by the target gas sensing member is used to determine the severity in five steps according to the concentration of the target gas, and control is performed accordingly. A first step is issuing a warning using a beacon or buzzer; a second step is triggering an emergency alarm linked with an indoor fire hydrant emergency bell; a third step is sending text notifications to an administrator and a fire station; a fourth step is forcibly shutting down a facility's power supply; and a fifth step is activating a fire extinguisher.

When multiple sensing members are used, the sensing location can be identified and displayed by using a MAC address or ID of the target gas sensing member, and text notifications can be sent to an administrator or the fire station.

### Industrial applicability

The fire prevention management system for a distribution board according to the present invention can be used in distribution boards in hospitals, power plants, substations, and factories where stable power supply is critical.

Since it detects diffusion of target gas before ignition, it is less affected by interference from obstacles and can manage fire prevention in real-time accurately and promptly.

## Claims

1. A fire prevention management system for a distribution board comprises;
a distribution board consisting of multiple metal enclosures;
a target gas sensing member installed inside a metal enclosure;
a monitoring member installed outside the metal enclosure that analyzes signals from the target gas sensing member; and
a communication member that, based on the analyzed concentration and occurrence location of a target gas, issues step-by-step alarms, sends signals, induces shutdown of an installed equipment, and transmits abnormal status.

2. The fire prevention management system as claimed in claim 1, wherein the target gas is a phthalate-based gas.

3. The fire prevention management system as claimed in claim 1, wherein the target gas is a carboxylate-based gas.

4. The fire prevention management system as claimed in claim 1, wherein the target gas sensing member includes a purified air injection member and an exhaust member that can detect real-time generated gas by continuously circulating air.

5. The fire prevention management system as claimed in claim 1, wherein the target gas sensing member is arranged in multiples within one metal enclosure at an equal target gas diffusion distance.

6. The fire prevention management system as claimed in claim 1, wherein a detection circuit of the target gas sensing member is equipped with a resistance heater to reduce errors and volatilize the target gas attached to a sensing resistor.

7. The fire prevention management system as claimed in claim 1, wherein the monitoring member determines a severity and controls it in the following steps:
a first step is to issue a warning using a warning light or buzzer;
a second step is to issue an emergency alarm by linking with the emergency bell of the indoor fire extinguisher;
a third step is to send a text message to a manager and fire department;
a fourth step is to forcibly cut off power to the equipment; and
a fifth step is to operate a fire extinguisher.

8. The fire prevention management system as claimed in claim 1, in cases where multiple target gas sensing members are used, the occurrence location of the target gas is identified by a MAC address of the target gas sensing member.

9. The fire prevention management system as claimed in claim, in cases where multiple target gas sensing members are used, the occurrence location of the target gas is identified by an ID of the target gas sensing member.
